# EUROPEAN PATENT SPECIFICATION

(11) **EP 3 761 026 B1**
(45) Date of publication and mention of the grant of the patent: **30.08.2023**
(21) Application number: 20183568.3
(22) Date of filing: 01.07.2020
(51) Int. Cl.: G01N 33/00, G01N 27/68

(54) **GAS HUMIDITY REDUCTION APPARATUS AND METHOD OF USING THE SAME**
VORRICHTUNG ZUR REDUKTION VON GASFEUCHTIGKEIT UND VERFAHREN ZUR VERWENDUNG DAVON
APPAREIL DE RÉDUCTION DE L'HUMIDITÉ DE GAZ ET SON PROCÉDÉ D'UTILISATION

(30) Priority: 04.07.2019 CN 201910598987
(43) Date of publication of application: 06.01.2021
(73) Proprietor: Honeywell International Inc., Morris Plains, NJ 07950 (US)
(72) Inventor: MENG, Zhongliang, Morris Plains, NJ New Jersey 07950 (US); ZHANG, Dan, Morris Plains, NJ New Jersey 07950 (US); XU, Yuhui, Morris Plains, NJ New Jersey 07950 (US); LU, Kui, Morris Plains, NJ New Jersey 07950 (US)
(74) Representative: Haseltine Lake Kempner LLP

(56) References cited:
- DE-A1- 4 010 482
- US-A- 4 231 256
- US-A- 5 250 093
- US-A- 5 457 316
- US-A1- 2015 362 468

## Description

### BACKGROUND

Industrial and commercial systems may use in-line gas detection systems to detect the presence of various gases within a gas flow path (e.g., an enclosed gas flow). A heightened humidity present within a gas flowing through a gas detection system may subject a gas sensor within the gas detection system to various undesirable conditions. For example, a photoionization detector (PID), when interacting with a volume of gas comprising a heightened relative humidity, may experience, among other effects, current leakage and/or a quenching effect. For example, when a PID experiences current leakage, an undesired amount of current leaks from between the electrodes in the sensor, often resulting in a false positive output signal. Further, a PID may undergo quenching in conditions of heightened relative humidity, wherein the water molecules within the PID block UV light from the gas of interest, resulting in reduced sensor responsiveness. These conditions affect the performance of the gas sensor, often leading to inaccurate sensor measurement, decreased measurement sensitivity, and/or other sensor failure conditions.

Accordingly, a need exists for solution system configured for reducing the humidity of a volume of gas flowing through a gas flow path, for example, to avoid damaging gas detection sensors.
DE4010482A1 discloses gas being cooled before delivery to a treatment unit or analyser. The gas is fed into a hollow filter body inside the cooling unit. In the filter cavity the gas pressure drops, with evolution of aerosols and mist. With pressure and temp. at normal levels, these materials are absorbed by the filter material, with the gas then leaving the cooler towards the test unit. US4231256A discloses a method and apparatus for removing moisture from a sample stream of gas while preventing dilution of the concentration of components of successive increments of the sample stream of gas.
US5457316A discloses an ion mobility spectrometer sensor apparatus which is in a hermetically sealed housing.
US5250093A discloses a water management system removes water vapor from the analyte slug that is desorbed from the trap.
US2015/362468A1 discloses a temperature and pressure regulating biogas sample extraction system and method for providing a conditioned biogas sample for constituent analysis.

### BRIEF SUMMARY

The invention is defined by claims 1 and 12.

In various embodiments, apparatus may further comprise a housing, which may comprise an exterior housing portion and an interior housing portion. In various embodiments, at least a portion of the gas delivery conduit, the at least one heat-conductive media, and the cooler are enclosed within the interior housing portion. In various embodiments, the at least one heat-conductive media may be configured to allow for the passage of a volume of gas therethrough. Further, in various embodiments, the at least one heat-conductive media may comprise a copper material. In various embodiments, the gas delivery conduit may comprise at least one conducting portion comprising heat-conductive sidewalls, and the at least one heat-conductive media may be positioned within an interior portion of the at least one conducting portion of the gas delivery conduit.

In various embodiments, the cooler may in contact with the at least one conducting portion of the gas delivery conduit. In various embodiments, the cooler may be embodied as a solid-state thermoelectric cooler, and may define a first side and a second side, wherein the first side may be configured as a cooling side and the second side may be configured as a heating side based at least in part on a voltage applied across the cooler. In various embodiments, at least a portion of the gas delivery conduit is in contact with the first side. In various embodiments, the apparatus may be configured to selectably reverse the voltage applied across the cooler such that the first side may configured as the heating side and the second side may configured as the cooling side. In various embodiments, the at least one heat-conductive media may comprise a first heat-conductive media and a second heat-conductive media positioned within an interior portion of a first conducting portion of the gas delivery conduit and a second conducting portion of the gas delivery conduit, respectively; the first conducting portion of the gas delivery conduit may be in contact with the first side of the cooler and the second conducting portion of the gas delivery conduit may be in contact with the second side of the cooler

In various embodiments, the apparatus may further comprise a pump configured to direct a flow of a volume of gas through the gas delivery conduit.

In various embodiments, the cooler may be a solid-state thermoelectric cooler and cooling the heat-conductive media may comprise applying a voltage in a first direction to the cooler to cool a first side of the cooler, wherein the first side of the cooler may be in contact with the at least one conducting portion of the gas delivery conduit. In various embodiments, the pump may be configured to change the directional flow of a volume of gas between a first flow direction and a second flow direction within the gas delivery conduit. In various embodiments, the apparatus may be configured to be fluidly connected to a photoionization detector and positioned upstream from the photoionization detector.

In various embodiments, the method may further comprise reversing the voltage applied to the cooler to heat the first side of the cooler to heat the at least one heat-conductive media to remove condensed water from the gas delivery conduit. Further, in various embodiments, the method may further comprise changing a directional flow of the volume of gas through the gas delivery conduit via a pump to a second flow direction opposite from the first flow direction. In various embodiments, the at least one conducting portion of the gas delivery conduit is fluidly connected to a photoionization detector and positioned upstream from the photoionization detector.

### BRIEF DESCRIPTION OF THE DRAWINGS

Figure 1 schematically illustrates an exemplary apparatus not according to the invention.
Figure 2 illustrates a cross-sectional view of an exemplary apparatus not according to the invention.
Figure 3 illustrates an exemplary apparatus in accordance with various embodiments of the invention.
Figure 4 illustrates a cross-sectional view of an exemplary apparatus as described herein.
Figure 5 illustrates an exemplary apparatus in accordance with various embodiments.
Figure 6 illustrates an exemplary apparatus in accordance with various embodiments.
Figure 7 shows an exemplary test configuration in accordance with various embodiments.
Figure 8 graphically illustrates the test results of the test configuration illustrated in Figure 7.
Figure 9 illustrates a flow diagram of an exemplary method for reducing the humidity of a volume of gas according to embodiments of the present disclosure.
Figure 10 illustrates a flow diagram of an exemplary method for reducing the humidity of a volume of gas according to embodiments of the present disclosure.
Figure 11 illustrates a flow diagram of an exemplary method for reducing the humidity of a volume of gas according to embodiments of the present disclosure.

### DETAILED DESCRIPTION

### Overview

Described herein is a method and apparatus for reducing the humidity of a volume of gas moving through a gas conduit, for example, prior to flowing the volume of gas through a gas detection sensor. In an example implementation, a humidity reduction apparatus as discussed herein is configured to utilize a cooling system to extract a volume of water from a volume of gas flowing through a gas conduit, thereby reducing the relative humidity of the gas. Such configurations are capable of reducing the humidity of a volume of gas while the volume of gas is flowing through a gas conduit.

As described herein, the humidity reduction apparatus may receive a volume of gas through a gas delivery conduit and, using a cooler, cool at least a portion of the gas delivery conduit in order to reduce the temperature of a volume of gas therein. As described herein, the cooler may be embodied as a solid-state (or Peltier-style) cooler having two opposing sides and may be configured such that when a voltage is applied across the cooler, heat is removed from a first side (thereby cooling the first side), and transferred to an opposite second side (thereby heating the second side). As described herein, one or both sides of the cooler may be in contact with respective portions of the gas delivery conduit. The humidity reduction apparatus as described herein may utilize a heat-conductive, porous media (e.g., a porous metal grid, a sintered metal filter, and/or the like) positioned within the gas delivery conduit to facilitate efficient heat transfer between the cooler and a volume of gas flowing through the gas delivery conduit by increasing the surface area of cooler-affected material within the conduit. Because the dwell time of gas flowing through the heat-conductive porous media is relatively short, the efficient heat-transfer effect of the porous media to the gas increases the effective cooling of the gas as it flows through the gas delivery conduit, thereby increasing the effectiveness of the humidity reduction effects as well.

In an exemplary implementation, a volume of gas may flow through a conductive portion of the gas delivery conduit having conductive sidewalls (e.g., metal sidewalls) in contact with a first, cooling side of the cooler. As described herein, the reduction of humidity within the volume of gas as it flows through a cooled porous heat-conductive material within the cooled conductive portion may result in an accumulation over time of liquid water condensate within the gas delivery conduit. In embodiments utilizing a solid-state cooler, the apparatus may be configured to reverse the voltage applied across the cooler, thereby heating the first side of the cooler, and by consequence, heating the previously cooled portion of the gas delivery conduit containing the accumulated condensate. When heated, a least a portion of the accumulated condensate evaporates, increasing the humidity of a gas flowing therethrough (e.g., air blown through the conduit during a clean-out cycle/process) such that the condensate may be removed from the gas delivery conduit.

### Humidity Reduction Apparatus

As disclosed herein a humidity reduction apparatus 10 may comprise a gas delivery conduit, at least one heat-conductive media positioned in-line within at least a portion of the gas delivery conduit, and a cooler to cool the gas delivery conduit and the at least one heat-conductive media.

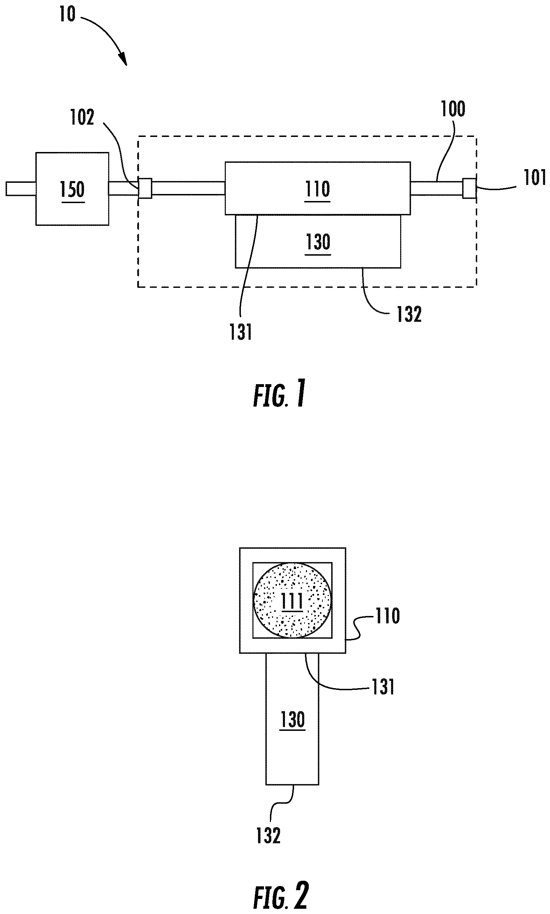

Figure 1 illustrates an exemplary humidity reduction apparatus 10, which is not according to the invention, and is positioned upstream from a gas detection sensor 150 comprising a gas delivery conduit 100. As shown, the gas delivery conduit of the humidity reduction apparatus 10 has an inlet 101 (for receiving gas flowing to the humidity reduction apparatus 10) and an outlet 102 (for directing gas flowing out of the humidity reduction apparatus 10) and may be configured to direct the flow of a volume of gas between the inlet 101 and the outlet 102. It should be understood that the humidity reduction apparatus 10 may be positioned in-line along a continuous length of gas delivery conduit, and accordingly the inlet 101 may be embodied as a portion of the continuous length of gas delivery conduit immediately adjacent (and upstream from) the cooler and the outlet 102 may be embodied as a portion of the continuous length of gas delivery conduit immediately adjacent (and downstream from) the cooler. The gas may travel in either a first flow direction or a second flow direction, wherein the second flow direction is opposite from the first flow direction. The humidity reduction apparatus 10 may be directional, such that the humidity reduction apparatus 10 is more effective for gas flowing in a first direction than for gas flowing in an opposite, second direction. However, it should be understood that certain examples may be reversible, such that the effectiveness of the humidity reduction apparatus 10 is not dependent of the direction of gas flow through the humidity reduction apparatus 10. The volume of gas may comprise one or more different types of gas.

The gas delivery conduit 100 may comprise at least one conducting portion. The at least one conducting portion may comprise heat-conductive sidewalls and a hollow interior portion so as to allow for the passage of a volume of gas therethrough. In various embodiments, the heat-conductive sidewalls of the at least one conducting portion may comprise a material having a high thermal conductivity. For example, the heat-conductive sidewalls may comprise copper, tin, aluminum, brass and/or other heat-conductive materials. As shown in Figure 1, the at least one conducting portion of the gas delivery conduit 100 may comprise a single conducting portion 110. The at least one conducting portion of the gas delivery conduit 100 may be linear, defining a single pass across a cooler 130. For example, as shown in Figure 1, the conducting portion 110 is linear, defining a single pass across the first side of the cooler 131.

As shown in the embodiment of Figure 1, the humidity reduction apparatus 10 further comprises a cooler 130. As shown, the cooler 130 is in contact with at least a portion of the conductive portion of the gas delivery conduit 100 so as to facilitate conductive heat transfer between the cooler 130 and the gas delivery conduit 100. The cooler 130 may be configured to selectively cool and/or heat said portion of the gas delivery conduit 100. In various embodiments, the cooler 130 may define a first side 131 and a second side 132. In various embodiments, at least a portion of the gas delivery conduit 100 may be in contact with the first side of the cooler 131. As illustrated in Figure 1, for example, the first side of the cooler 131 may be positioned at least substantially adjacent the conducting portion of the gas delivery conduit 110 such that the first side of the cooler 131 is in contact with a heat-conductive sidewall of the conducting portion 110. The cooler 130 may be either fixedly or removably attached to at least a portion of the gas delivery conduit 100. Although not shown, the conductive portion of the gas delivery conduit 110 may be secured relative to the first side of the cooler 130 via a heat-conductive adhesive (e.g., a silicone heat-transfer compound).

The cooler 130 may be embodied as, for example, a solid-state thermoelectric cooler. The cooler 130 may be configured to create a temperature difference between the first side 131 and the second side 132 based at least in part on a voltage applied across the cooler 130-a phenomenon known as the "Peltier effect." In various embodiments, a voltage may be applied across the cooler 130 such that one of the first side and the second side of the cooler 131, 132 is configured as a cooling side, while the other is configured as a heating side. For example, a voltage may be applied across the cooler 130 in a first direction such that the first side of the cooler 131 may be configured as a cooling side and the second side of the cooler 132 may be configured as a heating side. As shown in Figure 1, the first side of the cooler 131 may be configured to cool at least a portion of the gas delivery conduit 100. The first side of the cooler 131 may be configured to cool the conducting portion 110, thereby reducing the temperature of the heat-conductive sidewalls of the conducting portion 110 as well as the interior thereof (e.g., including a heat-conductive media 111 as described herein). A volume of humid gas present within the gas delivery conduit 100 may flow through the conducting portion 110 (which is cooled by the first side of the cooler 131) and the gas may experience a decrease in temperature. The decrease in temperature of the volume of gas may result in a volume of water condensing from the volume of gas, effectively reducing the humidity of the volume of gas. In various embodiments, the condensed water may collect within the interior portion of the conducting portion 110. The voltage applied to the solid-state thermoelectric cooler may be reversed, such that a voltage may be applied across the cooler 130 in a second direction to configure the first side of the cooler 131 as a heating side in order to heat the conducting portion 110, thereby increasing the temperature of both the heat-conductive sidewalls of the conducting portion 110 and the interior thereof. Such an exemplary configuration may cause at least a portion of the condensation present within the conducting portion 110 to evaporate into a flow of gas such that it may be removed from the apparatus through either the inlet 101 or outlet 102 of the gas delivery conduit 100 (e.g., during a cleanout process/cycle of operation of the humidity reduction apparatus).

The cooler 130 may be connected to a power supply configured to receive power and power the humidity reduction apparatus 10. As non-limiting examples, the power supply may comprise one or more batteries, one or more capacitors, one or more constant power supplies (e.g., a wall-outlet), and/or the like. The cooler 130 may be powered at between 1 and 12 volts (e.g., 5 volts). In various embodiments, a cooler 130 may comprise one or more of counter-flow shell-and-tube heat exchangers, plate heat exchangers (i.e. "plate chillers"), plate and shell heat exchangers, adiabatic wheel heat exchangers, plate fin heat exchangers, pillow plate heat exchangers, and/or the like.

Although not shown in Figure 1, the humidity reduction apparatus 10 may further comprise at least one heat-conductive media positioned in-line within at least a portion of the gas delivery conduit 100. Figure 2 illustrates a cross-sectional view of the exemplary humidity reduction apparatus 10 illustrated in Figure 1, wherein the at least one heat-conductive media comprises a single heat-conductive media 111. The at least one heat-conductive media may be configured to allow for the passage of a volume of gas therethrough. The at least one heat-conductive media may comprise a material having a high thermal conductivity, such as copper. As described herein, the at least one heat-conductive media may be configured to increase the surface area of heat-conductive material present within the gas delivery conduit 100. Accordingly, the heat-conductive media may be embodied as a highly porous sintered metal filter, a grid, a porous screen, and/or the like. In certain embodiments, the heat-conductive media may have a high surface area for interaction with gas passing through the heat-conductive media, while minimizing the pressure drop of fluid flow across the heat-conductive media. Moreover, it is believed that the heat-conductive media may additionally create a turbulent flow of gas through the heat-conductive media, thereby further facilitating conductive and/or convective heat transfer between the heat-conductive media and the gas flowing therethrough.

As shown in Figure 2, an example not according to the invention, the heat-conductive media 111 may be positioned within an interior portion of the conducting portion of the gas delivery conduit 110 and may be in contact with an interior surface of sidewalls of the conducting portion of the gas delivery conduit 110 to facilitate conductive heat transfer between the sidewalls of the conducting portion of the gas delivery conduit 110 and the heat-conductive media 111.

For example, the heat-conductive media 111 may be welded to the interior of the conducting portion of the gas delivery conduit 110, may be adhered (e.g., via a heat-conductive adhesive) within the gas delivery conduit 110, and/or the like. Further, as described above with respect to Figure 1, the first side of the cooler 131 may be in contact with conducting portion 110. In various embodiments, the first side of the cooler 131 may be configured to cool the conducting portion 110 and the included heat-conductive media 111, for example, via conductive heat transfer. The heat-conductive media 111 may be configured to facilitate a temperature decrease within the interior of conducting portion 110. Further, the heat-conductive media 111 may increase the surface area of heat-conductive material conductively connected to the cooler 130, thereby increasing the heat-transfer between a volume of gas flowing through the gas delivery conduit 100 and the cooler. Accordingly, as described herein, a volume of humid gas flowing through the gas delivery conduit 100 may flow through the conducting portion 110 and the heat-conductive media 111 cooled by the first side of the cooler 131, which may cause the temperature of the gas flowing through the conducting portion 110 to reduce thereby leading to a decrease in humidity within the gas. A volume of water condensed from the volume of gas may collect within the heat-conductive media 111 and/or the conductive portion of the gas delivery conduit 110.

As described above with respect to Figure 1, the cooler 130 may be configured to selectively heat the conductive portion of the gas delivery conduit 110 to evaporate condensed water therein (e.g., during a clean-out process/cycle) to ease removal of the collected condensate from the gas delivery conduit 110. For example, a voltage may be applied across the solid-state thermoelectric cooler 130 in a second direction such that the first side of the cooler 131 may be configured as a heating side in order to heat the conducting portion 110, thereby increasing the temperature of the interior portion and the heat-conductive sidewalls of the conducting portion 110, as well as the heat-conductive media 111. Such an exemplary configuration may cause at least a portion of the condensation present within heat-conductive media 111 to evaporate such that it may be removed from the apparatus through either the inlet 101 or outlet 102 of the gas delivery conduit 100.

It should be understood that, although the conducting portion of the gas delivery conduit 110 is illustrated as having a substantially rectangular cross section, any of the at least one conducting portions may have a cross-section of any shape and may comprise any number of sidewalls suitable for operation of the humidity reduction apparatus 10 as described herein. Similarly, it should be understood that, although the heat-conductive media 111 is illustrated as having a substantially round cross section, any of the at least one heat-conductive media may have a cross-section of any shape and may comprise any number of sidewalls suitable for operation of the humidity reduction apparatus 10 as described herein. The heat-conductive media 111 may fill the interior of the conducting portion of the gas delivery conduit 110, such that the length and the cross-sectional area of the porous heat-conductive media 111 are at least substantially equal to the length and the cross-sectional area of the interior of the conducting portion of the gas delivery conduit 110, respectively. The cross-sectional shape of the porous heat-conductive media 111 may correspond to (e.g., match) the cross-sectional shape of the conducting portion of the gas delivery conduit 110. For example, the conducting portion of the gas delivery conduit 110 may have a circular cross-section and the porous heat-conductive media 111 may have a corresponding circular cross-section. However, as described herein the cross-sectional shape of the porous heat-conductive media 111 need not correspond to the cross-sectional shape of the conducting portion of the gas delivery conduit 110. For example, the conducting portion of the gas delivery conduit 110 may have a square or rectangular cross-section, and the porous heat-conductive media 111 may have a circular cross-sectional shape. Further, in various embodiments, the length of the porous heat-conductive media 111 need not correspond to the length of the interior of the conducting portion of the gas delivery conduit 110. For example, the porous heat-conductive media 111 may have a length that is less than that of the interior of the conducting portion of the gas delivery conduit 110 such that the porous heat-conductive media 111 does not extend along the entirety of the length of the interior of the conducting portion of the gas delivery conduit 110 along the fluid flow path of the gas delivery conduit 100.

As shown in Figure 1, a gas detection sensor 150 may be positioned downstream from the humidity reduction apparatus 10 and may be fluidly connected to the gas delivery conduit outlet 102 such that gas exiting the gas delivery conduit outlet 102 enters an inlet of the gas detection sensor 150. The gas detection sensor 150 may comprise, for example, one of an electrochemical gas sensor, a catalytic gas sensor, a solid-state gas sensor, a non-dispersive infrared gas sensor (NDIR), a PID, and/or the like. For example, the humidity reduction apparatus 10 may be fluidly connected to and positioned upstream from a PID so as to reduce the humidity of a volume of before the gas is evaluated by the PID.

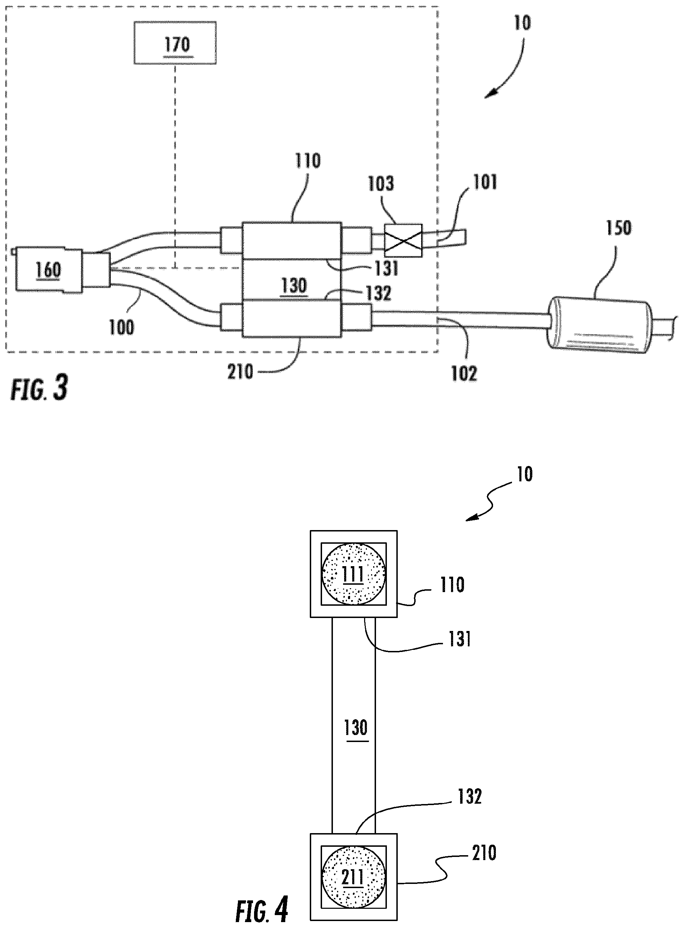

Figure 3 illustrates a humidity reduction apparatus 10 according to the invention, wherein the at least one conducting portion of the gas delivery conduit 100 comprises a first conducting portion 110 and a second conducting portion 210. In various embodiments, both the first conducting portion 110 and the second conducting portion 210 may be in contact with the cooler 130 so as to facilitate conductive heat transfer between the cooler 130 and the respective conducting portions 110, 210. In various embodiments wherein the humidity reduction apparatus 10 is configured such that a volume of gas travels along the gas delivery conduit in a first flow direction, as described herein, the first conducting portion 110 may be upstream from the second conducting portion 210. In various embodiments, the gas delivery conduit 100 may be non-linear, defining one or more passes across a cooler. As shown in Figure 3, the gas delivery conduit is non-linear, defining two passes across the cooler 130.

As shown in Figure 3, the first conducting portion 110 may be in contact with a first side of the cooler 131 while a second conducting portion 210 may be in contact with a second side of the cooler 132. In various embodiments, as described herein, a voltage may be applied across the cooler 130 in a first direction to configure the first side of the cooler 131 as a cooling side and the second side of the cooler 132 as a heating side. The first side of the cooler 131 may be configured to cool at least a portion of the gas delivery conduit 100, while the second side of the cooler 132 may be configured to heat at least a portion of the gas delivery conduit 100. As shown in Figure 3, the first side of the cooler 131 may be configured to cool the first conducting portion 110, thereby reducing the temperature of the heat-conductive sidewalls of the first conducting portion 110 as well as the interior thereof (e.g., including a first heat-conductive media 111 as described herein). Conversely, the second side of the cooler 132 may be configured to heat the second conducting portion 210, thereby increasing the temperature of the heat-conductive sidewalls of the second conducting portion 210 as well as the interior thereof (e.g., including a second heat-conductive media 211 as described herein). In such an exemplary configuration, and further when the humidity reduction apparatus 10 is configured such that a volume of gas travels along the gas delivery conduit in a first flow direction, the first conducting portion 110 (which is cooled by the first side of the cooler 131) may be upstream from the second conducting portion 210 (which is heated by the second side of the cooler 132).

Although not shown in Figure 3, the one or more heat-conductive media of the humidity reduction apparatus 10 comprises a first heat-conductive media 111 and a second heat-conductive media 211. As shown in Figure 4, the first heat-conductive media 111 and the second heat-conductive media 211 are positioned within an interior portion of a first conducting portion of the gas delivery conduit 110 and a second conducting portion of the gas delivery conduit 210, respectively. Further, the first heat-conductive media 111 and the second heat-conductive media 211 may be in contact with an interior surface of sidewalls of the first and second conducting portions of the gas delivery conduit 110, 210, respectively, to facilitate conductive heat transfer between the sidewalls of the first and second conducting portions of the gas delivery conduit 110, 210 and the first and second heat-conductive media 111, 211, respectively. The first heat-conductive media 111 may fill the interior of the first conducting portion of the gas delivery conduit 110, such that the cross-sectional area of the heat-conductive media 111 is at least substantially equal to the cross-sectional area of the interior of the first conducting portion of the gas delivery conduit 100. Similarly, The second heat-conductive media 211 may fill the interior of the second conducting portion of the gas delivery conduit 210, such that the cross-sectional area of the second heat-conductive media 211 is at least substantially equal to the cross-sectional area of the interior of the second conducting portion of the gas delivery conduit 100. In such embodiments, the respective cross-sectional shapes of the first and second heat-conductive media 111 may correspond to (e.g., match) the cross-sectional shapes of the first and second conducting portions of the gas delivery conduit 110, 210, respectively. For example, the first conducting portion of the gas delivery conduit 110 may have a circular cross-section and the first heat-conductive media 111 may have a corresponding circular cross-section. However, in other embodiments, the respective cross-sectional shapes of the first and second heat-conductive media 111, 211 need not correspond to the cross-sectional shape of the first and second conducting portion 110, 210. For example, the second conducting portion of the gas delivery conduit 210 may have a square or rectangular cross-section, and the second heat-conductive media 211 may have a circular cross-sectional shape.

For example, the first and second heat-conductive media 111, 211 may be welded to the interior of the first and second conducting portion of the gas delivery conduit 110, 210, respectively, may be adhered (e.g., via a heat-conductive adhesive) within the first and second conducting portions, 110, 210, respectively, and/or the like. Further, as described herein, the first conducting portion 110 may be in contact with a first side of a cooler 131, while a second conducting portion 210 may be in contact with a second side of a cooler 132.

In various embodiments, based at least in part on a voltage applied across the cooler 130 as described herein, the first side of the cooler 131 may be configured to cool the first conducting portion 110 and the included first heat-conductive media 111, while the second side of the cooler 132 may be configured to heat the second conducting portion 210 and the included second heat-conductive media 211, for example, via conductive heat transfer.

Alternatively, in various embodiments, the voltage applied to the solid-state thermoelectric cooler may be reversed, such that a voltage may be applied across the cooler 130 in a second direction to configure the first side of the cooler 131 as a heating side and the second side of the cooler 132 as a cooling side. In such a configuration, the first side of the cooler 131 may be configured to heat at least a portion of the gas delivery conduit 100, while the second side of the cooler 132 may be configured to cool at least a portion of the gas delivery conduit 100. As shown in Figure 3, when a voltage is applied across the cooler 130 in the second direction, the second side of the cooler 132 may be configured to cool the second conducting portion 210, thereby reducing the temperature of the heat-conductive sidewalls of the second conducting portion 210 as well as the interior thereof (e.g., including a second heat-conductive media 211 as described herein). Conversely, the first side of the cooler 131 may be configured to heat the first conducting portion 110, thereby increasing the temperature of the heat-conductive sidewalls of the first conducting portion 110 as well as the interior thereof (e.g., including a first heat-conductive media 111 as described herein). In various embodiments, a volume of humid gas present within the gas delivery conduit 100 may flow through the second conducting portion 210 (which is cooled by the second side of the cooler 132) and the gas may experience a decrease in temperature. In such an exemplary embodiment, the decrease in temperature of the volume of gas may result in a volume of water condensing from the volume of gas, effectively reducing the humidity of the volume of gas. In various embodiments, the condensed water may collect within the interior portion of the second conducting portion 210.

In various embodiments, the humidity reduction apparatus may further comprise a pump 160 configured to direct the flow of a volume of gas through the gas delivery conduit 100. As shown in Figure 3, the pump 160 may be positioned in-line with the gas delivery conduit 100. In various embodiments, the pump 160 may be configured to change the directional flow of a volume of gas between a first flow direction and a second flow direction within the gas delivery conduit. In various embodiments, a first flow direction may extend along the gas delivery conduit 100 from the inlet 101 towards the outlet 102, and a second flow direction may extend along the gas delivery conduit 100 from the outlet 102 towards the inlet 101. In various embodiments, the directional flow of a volume of gas within the gas delivery conduit 100 may be changed from a first flow direction to a second flow direction, as described herein, by providing power to the pump 160 where the pump 160 was previously unpowered. Alternatively, the directional flow of a volume of gas within the gas delivery conduit 100 may be changed between a first flow direction and a second flow direction, as described herein, by reversing the polarity of a pump input signal. For example, in a configuration wherein a volume of gas is directionally flowing in a first flow direction such that the outlet 102 is downstream from the inlet 101, the pump 160 may be configured to reverse the directional flow of the volume of gas from the first flow direction to the second flow direction such that the outlet 102 is upstream from the inlet 101 based on a reversal of one or more pump input signals.

In some embodiments, the pump 160 may be connected to a power supply configured to receive power and power the humidity reduction apparatus 10. As non-limiting examples, the power supply may comprise one or more batteries, one or more capacitors, one or more constant power supplies (e.g., a wall-outlet), and/or the like. In various embodiments, the pump 160 may be powered at between 1 and 12 volts (e.g., 5 volts).

In various embodiments, the humidity reduction apparatus may further comprise a controller 170. In various embodiments, the controller 170 may be configured for distribution of power to a cooler 130, one or more additional coolers, and/or a pump 160 as described herein. In some embodiments, a cooler 130 and/or a pump 160 may be connected to controller 170 (e.g., for electronic communication), which may be configured to facilitate functional control therebetween. In various embodiments, the controller 170 may comprise at least a processor and/or a memory (e.g., non-transitory memory). In another example, the memory may be a non-transitory computerreadable storage medium storing computer-executable program code instructions that, when executed by a computing system, cause the computing system to perform the various operations described herein. The memory may be configured to store information, data, content, signals, applications, instructions (e.g., computer-executable program code instructions), or the like, for enabling the controller 170 to carry out various functions in accordance with example embodiments of the present disclosure. In various embodiments, for example, the processor may be configured to control voltage direction across the cooler 130, control a pump 160, or execute one or more humidity reduction cycles for the humidity reduction apparatus 10. For example, in various embodiments, the processor may be configured to change the voltage direction across a cooler 130 from a first direction to a second direction and from a second direction to a first direction. In various embodiments, the processor may be configured to control the power state and directional configuration of a pump 160. In various embodiments, the processor may be configured to execute various instructions related to a heat reduction cycle, such as, for example, instructions to change voltage direction across the cooler 130, or control the power state and/or directional configuration of the pump 160, in order to cool at least a portion of the gas delivery conduit and subsequently remove at least a portion of an accumulated condensate from the gas delivery conduit 100 as described herein.

In various embodiments, the humidity reduction apparatus may further comprise a valve assembly 103, which may be configured to remove at least a portion of accumulated condensate from within the gas delivery conduit. In various embodiments, the valve assembly 103 may comprise a valve positioned along the gas delivery conduit 100. In various embodiments, the valve assembly 103 may be positioned either upstream or downstream from the at least one conducting portion of the gas delivery conduit 100. In various exemplary implementations, such as, for example, during a clean-out process/cycle, the valve may be configured to allow a volume of accumulated condensate to dispense from the gas delivery conduit. For example, as shown in Figure 3, during a clean-out process/cycle wherein air is flowing through the gas delivery conduit 100 in a second fluid flow direction, the valve assembly 103 may be configured to dispense a volume of accumulated condensate traveling from the conducting portion 110 to the gas delivery conduit inlet 101 out of the gas delivery conduit via a valve. In various embodiments, the valve assembly 103 may further comprise a storage tank fluidly connected to the valve and configured to receive and store a volume of water dispensed from the gas delivery conduit 100 via the valve.

Figure 4 illustrates a cross-sectional view of components of the exemplary humidity reduction apparatus 10 illustrated in Figure 3. As shown in Figure 4 and discussed herein, the at least one heat-conductive media may comprise a first heat-conductive media 111 and a second heat-conductive media 211 positioned in-line within at least a portion of the gas delivery conduit 100. In various embodiments, the first and second heat-conductive media 111, 211 may comprise either the same or different material and may exhibit either the same or different material properties. In various embodiments, the first and second heat-conductive media 111, 211 may be configured to facilitate a temperature change of a volume of gas flowing through a conductive portion of the gas delivery conduit 110, 210.

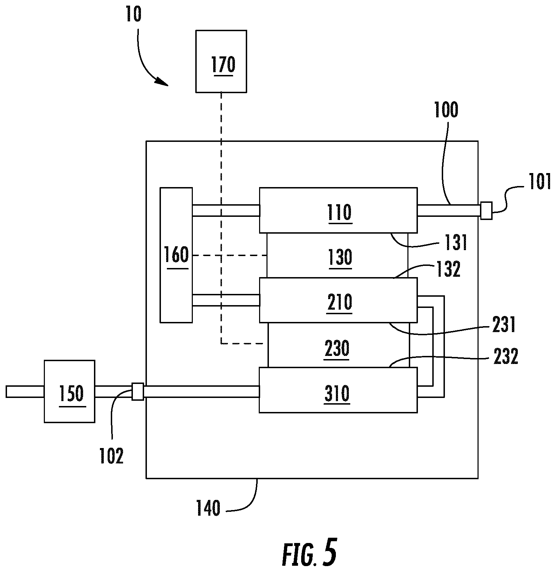

Figure 5 illustrates an exemplary humidity reduction apparatus 10 wherein the at least one conducting portion of the gas delivery conduit 100 comprises a first conducting portion 110, a second conducting portion 210, and a third conducting portion 310, and wherein the exemplary humidity reduction apparatus 10 further comprises a second cooler 230. As shown in Figure 5, the cooler 130, the first heat-conductive portion of a gas delivery conduit 110, and the second heat-conductive portion of the gas delivery conduit 210 are collectively configured in the same configuration as described above with respect to the exemplary embodiment illustrated in Figures 3 and 4.

In various embodiments, the humidity reduction apparatus 10 may comprise one or more additional coolers in addition to the cooler 130. In various embodiments, the one or more additional coolers may be in contact with at least a portion of the gas delivery conduit 100 and may be configured to selectively cool and/or heat said portion of the gas delivery conduit 100. As shown in Figure 5, the one or more additional coolers comprise a second cooler 230. In various embodiments, the second cooler 230 may define a first side 231 and a second side 232. In various embodiments, at least a portion of the gas delivery conduit 100 may be in contact with both the first side of the second cooler 231 and the second side of the second cooler 232. As illustrated in Figure 5, for example, the first side of the second cooler 231 may be positioned at least substantially adjacent a second conducting portion of the gas delivery conduit 210 such that the first side of the second cooler 231 is in contact with a heat-conductive sidewall of the second conducting portion 210, while the second side of the second cooler 232 may be positioned at least substantially adjacent a third conducting portion 310 such that the second side of the second cooler 232 is in contact with a heat-conductive sidewall of the third conducting portion 310. In such an exemplary configuration, the first and second ends of the second cooler 231, 232 may be conductively connected to the second and third conducting portion 210, 310, respectively, to facilitate a heat transfer between the components. In various embodiments, the second cooler 230 may be either fixedly or removably attached to at least a portion of the gas delivery conduit 100.

In various embodiments, the second cooler 230 may comprise a solid-state thermoelectric cooler and may be configured to operate and function in a manner similar to that of cooler 130. In various embodiments, as described herein with respect to cooler 130, a voltage may be applied across the cooler 130 such that one of the first side and the second side of the second cooler 231, 232 is configured as a cooling side, while the other is configured as a heating side. For example. In various embodiments, the one or more additional coolers may comprise a type of cooler other than a solid-state thermoelectric cooler.

In various embodiments, as shown in Figure 5, the humidity reduction apparatus 10 may further comprise a housing 140. The housing 140 may comprise an exterior housing portion and an interior housing portion. In various embodiments, at least a portion of the gas delivery conduit 100, the at least one heat-conductive media, and the cooler 130 may be enclosed within the interior housing portion of the housing 140. Further, in various embodiments, one or more of the pump 160, the controller 170, and one or more additional coolers may also be enclosed within the interior housing portion of the housing 140. As shown in Figure 5, the cooler 130, the second cooler 230, at least a portion of the gas delivery conduit 100, including the first, second, and third conducting portions of the gas conduit 110, 210, 310, and the pump 160 are each enclosed within the interior housing portion of the housing 140.

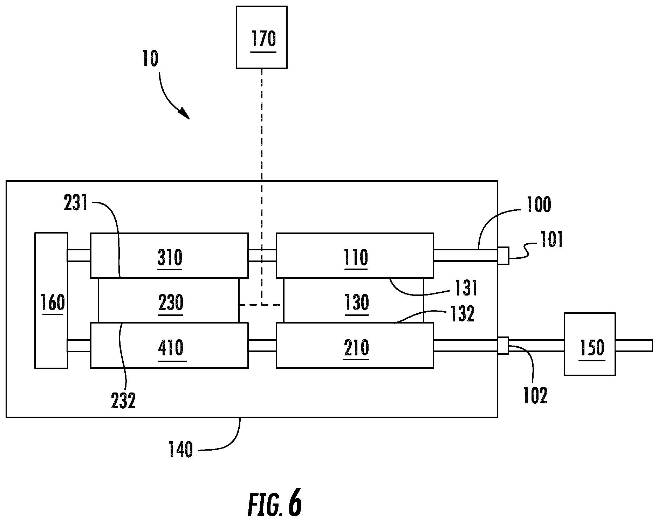

Figure 6 illustrates an exemplary humidity reduction apparatus 10 wherein the at least one conducting portion of the gas delivery conduit 100 comprises a first conducting portion 110, a second conducting portion 210, a third conducting portion 310, and a fourth conducting portion 410, and wherein the one or more additional coolers of the humidity reduction apparatus 10 as described herein comprises a second cooler 230. As shown in Figure 6, the cooler 130, the first heat-conductive portion of a gas delivery conduit 110, and the second heat-conductive portion of the gas delivery conduit 210 are collectively configured in the same configuration as described above with respect to the exemplary embodiment illustrated in Figures 3 and 4.

As illustrated in Figure 6, for example, the first side of the second cooler 231 may be positioned at least substantially adjacent a third conducting portion of the gas delivery conduit 310 such that the first side of the second cooler 231 is in contact with a heat-conductive sidewall of the third conducting portion 310, while the second side of the second cooler 232 may be positioned at least substantially adjacent a fourth conducting portion 410 such that the second side of the second cooler 232 is in contact with a heat-conductive sidewall of the fourth conducting portion 410. In such an exemplary configuration, the first and second ends of the second cooler 231, 232 may be conductively connected to the third and fourth conducting portions 310, 410, respectively, to facilitate a heat transfer between the components.

### Experimental Testing and Results

Experimental testing was conducted to verify the effectiveness of embodiments as described herein. Data was collected over the course of multiple trials using various combinations of embodiments described above.

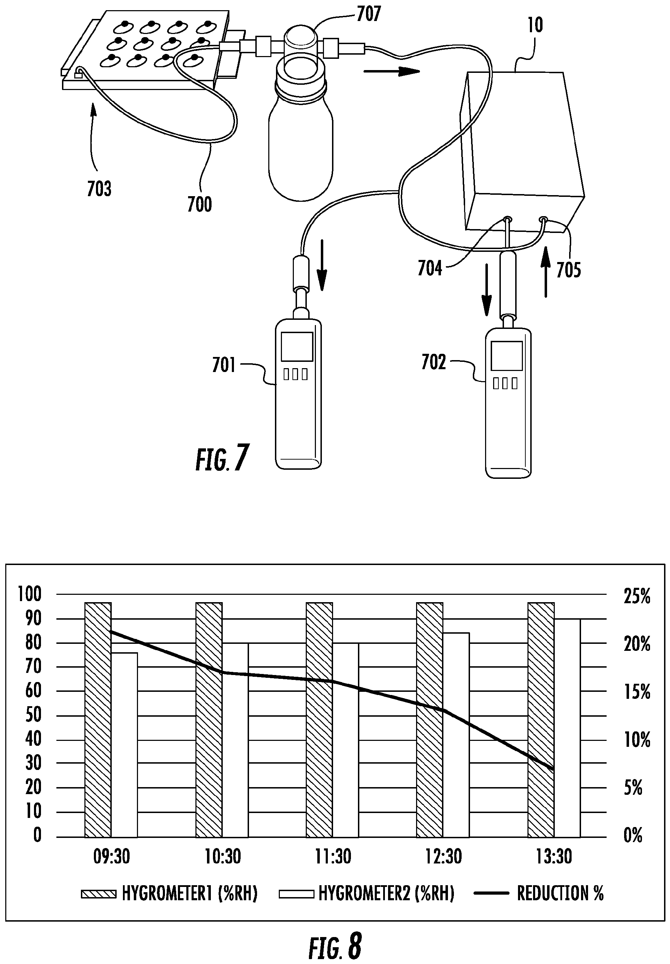

Figure 7 shows the components of the humidity reduction apparatus 10 used in the exemplary testing configuration. A testing pump 703 was used to drive the flow of a volume of gas through a preliminary portion of a gas delivery conduit 700 to the gas delivery conduit inlet 705. Prior to reaching the gas delivery conduit inlet 705, the volume of gas was exposed to a chamber at least partially filled with a volume of water 707 so as to artificially manufacture a relative humidity of at least substantially 100% within the volume of gas. A hygrometer 701 was positioned upstream from the gas delivery conduit inlet 705 in order to measure the relative humidity of the volume of gas before entering the humidity reduction apparatus 10. Further, a hygrometer 702 was positioned downstream from the gas delivery conduit outlet 704 in order to measure the relative humidity of the volume of gas after it passed through the humidity reduction apparatus 10.

The humidity reduction apparatus 10 as used in the exemplary testing configuration comprised a single solid-state thermoelectric cooler, a first heat-conductive media positioned inline within a first conducting portion of the gas delivery conduit and a second heat-conductive media positioned in-line within a second conducting portion of the gas delivery conduit. The aforementioned components were configured in a similar manner to those of the exemplary embodiment illustrated in Figures 3 and 4, wherein the gas delivery conduit defined a non-linear fluid flow path and defined two passes across the cooler at a first conducting portion and a second conducting portion. In the exemplary testing embodiment, the exemplary cooler was configured to cool the first conducting portion of the gas delivery conduit and the first heat-conductive media positioned therein. The first conducting portion of the gas delivery conduit and the first heat-conductive media positioned therein were positioned upstream from the second conducting portion of the gas delivery conduit and the second heat-conductive media positioned therein.

Figure 8 shows a graphical representation of the data collected in various experimental trials of the embodiments of the claimed inventions. Figure 8 graphically illustrates a comparison of two measured data points: the relative humidity of a volume of gas before it entered the exemplary humidity reduction apparatus-represented by the measurements of hygrometer 1-to the relative humidity of the volume of gas after it passed through the exemplary apparatus-represented by the measurements of hygrometer 1. The two aforementioned data points were each collected at five distinct instances, starting at 9 hours and 30 minutes after the exemplary apparatus began running (i.e. a voltage began flowing across the cooler) and subsequently every 60 minutes for the next four hours. Figure 8 further graphically illustrates the reduction percentage between the two hygrometer measurements; that is, the percentage decrease in the relative humidity of the volume of gas after having passed through the exemplary testing apparatus. As shown in Figure 8, the relative humidity percentage is measured along the primary (left) y-axis and the calculated relative humidity reduction percentage is measured along the secondary (right) y-axis. Data collected at each of the five time intervals are distinctly arranged in chronological order from left to right along the x-axis. Figure 8 graphically represents the data shown in Table 1 below:

**TABLE 1**

| Time | Hygrometer 1 (%RH) | Hygrometer2(%RH) | Reduction% |
|---|---|---|---|
| 09:30 | 96.5 | 76 | 21% |
| 10:30 | 96.8 | 80.2 I | 17% |
| 11:30 | 96.5 | 80.6 I | 16% |
| 12:30 | 96.5 | 84.3 I | 13% |
| 13:30 | 96.5 | 89.6 I | 7% |

As illustrated by Figure 8, the inlet relative humidity (i.e. the measured humidity at hygrometer 1) was maintained at around 96.5% throughout the test in order to simulate a worstcase condition. Notably, at each data collection instance, the exemplary humidity reduction apparatus 10 testing configuration successfully reduced the relative humidity of the measured volume of gas. The measured reduction in relative humidity percentage between the first and second hygrometers ranged between 5% and 21%. It should be understood that the specific testing results illustrated in Figure 8 and described herein are a product of the exemplary humidity reduction apparatus 10 testing configuration as described herein. In various embodiments, the relative humidity reduction percentage may either increase or decrease based on various testing configuration variables, such as, for example, the inlet relative humidity, the configuration of the humidity reduction apparatus 10, and the amount of time between when the apparatus was turned on and when the data was collected.

### Method

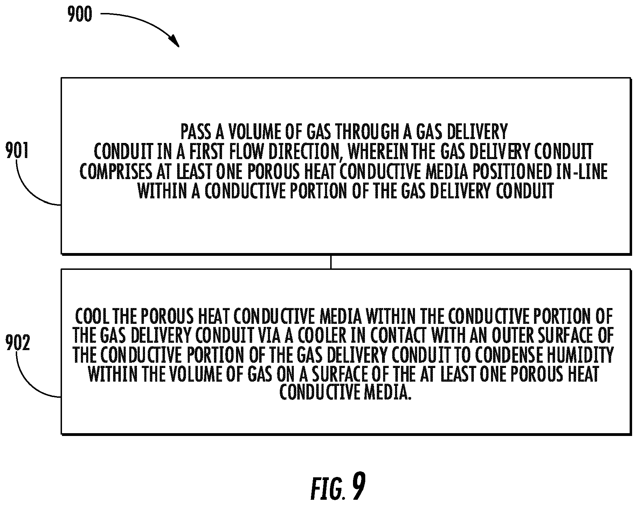

Figure 9 illustrates a block diagram of an exemplary method 900 for reducing the humidity of a volume of gas in accordance with some embodiments discussed herein.

At block 901, a volume of gas is passed through a gas delivery conduit in a first flow direction. The gas delivery conduit comprises at least one conducting portion, and at least one heat-conductive media is positioned in-line within the at least one conductive portion of the gas delivery conduit. In various embodiments, a first flow direction may extend along the gas delivery conduit from an inlet towards an outlet.

Further, at block 902, the at least one heat-conductive media is cooled via a cooler in contact with an outer surface of the conductive portion of the gas delivery conduit to condense humidity within a volume of gas on surfaces of the at least one heat-conductive media. In various embodiments, the cooler may be a solid-state thermoelectric cooler and cooling the heat-conductive media may comprise applying a voltage in a first direction to the cooler to cool a first side of the cooler, wherein the first side of the cooler is in contact with the at least one conducting portion of the gas delivery conduit.

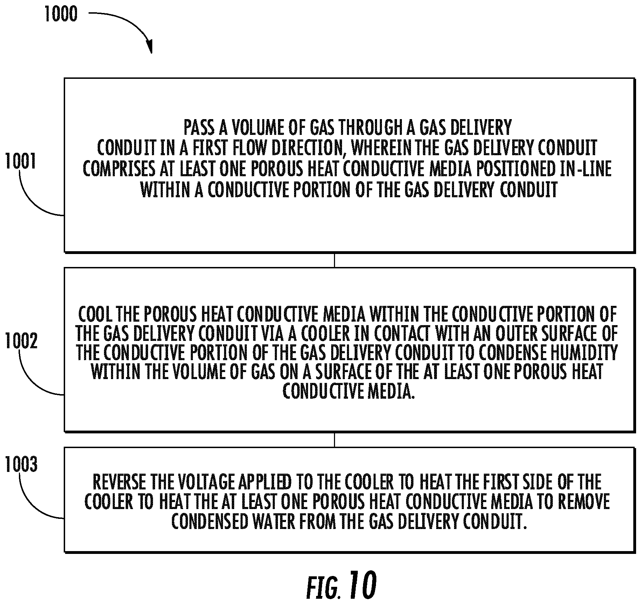

Figure 10 illustrates a block diagram of an exemplary method 1000 for reducing the humidity of a volume of gas in accordance with some embodiments discussed herein. Blocks 1001 and 1002 of the exemplary method 1000 are at least substantially similar to the steps previously disclosed at blocks 901 and 902, respectively.

At block 1003, the exemplary method 1000 further comprises reversing the voltage applied to the cooler to heat the first side of the cooler to heat the at least one heat-conductive media to remove condensed water from the gas delivery conduit. In various embodiments, as one or more volumes of gas pass through the at least one heat-conductive media over time, the condensate extracted from the one or more volumes of gas may begin to accumulate. After a period of time, the accumulated condensate on the surfaces of the at least one heat-conductive media, will accumulate to such an extent that the volume of condensate present within the gas delivery conduit will begin to negatively impact the reduction of humidity described herein. In various embodiments, the negative effects of accumulated condensate within the gas delivery conduit and amount of time before which said negative effects may be realized may depend on one or more variables, such as, for example, the flow rate and relative humidity of a volume of gas passing through the gas delivery conduit. For example, in various embodiments, an accumulated condensate within a gas delivery conduit may begin to negatively impact the reduction of humidity of a volume of gas between 2 and 10 hours (e.g., 8 hours) after the cooler has begun cooling the volume of gas. Accordingly, the voltage applied to the cooler may be reversed from a first direction to a second direction so as to switch the configuration of the first side of the cooler from a cooling configuration to a heating configuration, as described herein. Upon heating the at least one conducting portion of the gas delivery conduit, and thus to the at least one heat-conductive media positioned therein, at least a portion of the accumulated condensate may evaporate. The evaporated condensate (i.e. steam) may be removed from the gas delivery conduit via the flow of a volume of gas.

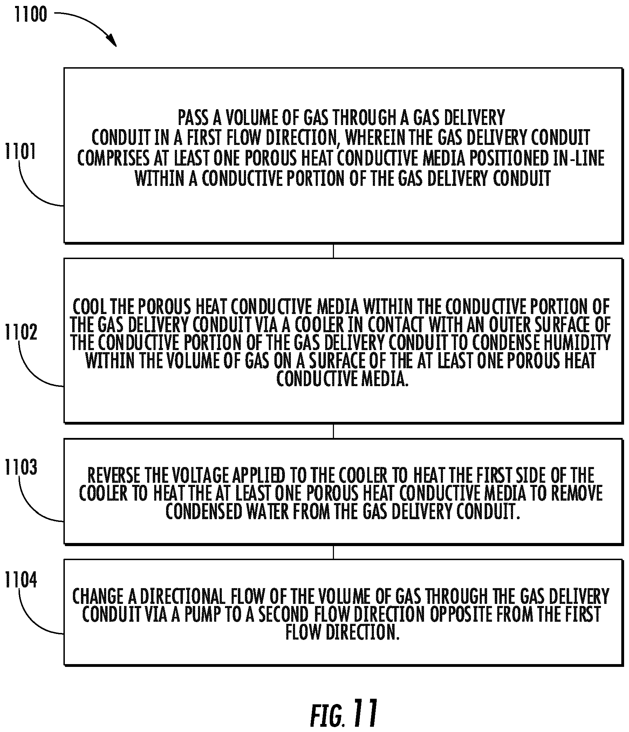

Figure 11 illustrates a block diagram of an exemplary method 1100 for reducing the humidity of a volume of gas in accordance with some embodiments discussed herein. Blocks 1101, 1102, and 1103 of the exemplary method 1100 are at least substantially similar to the steps previously disclosed at blocks 1001, 1002, and 1003, respectively.

At block 1103, the exemplary method 1100 further comprises changing a directional flow of the volume of gas through the gas delivery conduit via a pump to a second flow direction opposite from the first flow direction. In various embodiments, a second flow direction may extend along the gas delivery conduit from an outlet towards an inlet. In such an exemplary configuration, the evaporated condensate (i.e. steam) may be removed from the gas delivery conduit via the inlet by flowing in the second flow direction. In various embodiments, the directional flow of a volume of gas within the gas delivery conduit may be changed to a second flow direction, as described herein, by, for example, providing power to a pump where the pump was previously unpowered or reversing the polarity of a pump input signal.

## Claims

1. An apparatus (10) for reducing the humidity of a volume of gas, the apparatus comprising:
a gas delivery conduit (100) and comprising a first conducting portion (110) and a second conducting portion (210);
at least one heat-conductive media comprising a first heat-conductive media (111) positioned in-line within the first conducting portion (110) portion of the gas delivery conduit (100) and a second heat-conductive media (211) positioned in-line within the second conducting portion (210) of the gas delivery conduit (100); and
a cooler (130) in contact with the first conducting portion (110) and the second conducting portion (210) of the gas delivery conduit (100) to cool the first conducting portion (110) and the second conducting portion (210) of the gas delivery conduit and the first and second heat-conductive media.

2. The apparatus (10) of Claim 1, further comprising a housing (140), wherein the housing comprises an exterior housing portion and an interior housing portion, and wherein at least a portion of the gas delivery conduit (100), the at least one heat-conductive media, and the cooler (130) are enclosed within the interior housing portion.

3. The apparatus (10) of Claim 1, wherein the at least one heat-conductive media is configured to allow for the passage of a volume of gas therethrough.

4. The apparatus (10) of Claim 1, wherein the at least one heat-conductive media comprises a copper material.

5. The apparatus (10) of Claim 1, wherein the first and second conducting portions (110,210) comprise heat-conductive sidewalls, and wherein first and second heat-conductive media are positioned within an interior portion of the first and second conducting portions of the gas delivery conduit (100).

6. The apparatus (10) of Claim 1, wherein the cooler (130) is embodied as a solid-state thermoelectric cooler.

7. The apparatus (10) of Claim 6, wherein the cooler (130) defines a first side (131) and a second side (132), and wherein the first side (131) is configured as a cooling side and the second side (132) is configured as a heating side based at least in part on a voltage applied across the cooler (130), and wherein the at least a portion of the gas delivery conduit (100) is in contact with the first side.

8. The apparatus (10) of Claim 7, wherein the apparatus (10) is configured to selectably reverse the voltage applied across the cooler (130) such that the first side (131) is configured as the heating side and the second side (132) is configured as the cooling side.

9. The apparatus (10) of Claim 8, wherein the first heat-conductive media (111) and a second heat-conductive media (211) are positioned within an interior portion of the first conducting portion of the gas delivery conduit (110) and the second conducting portion of the gas delivery conduit (210), respectively, and wherein the first conducting portion of the gas delivery conduit (210) is in contact with the first side of the cooler (131) and the second conducting portion of the gas delivery conduit (210) is in contact with the second side of the cooler (132).

10. The apparatus (10) of Claim 1, further comprising a pump (160) configured to direct a flow of a volume of gas through the gas delivery conduit (100) and change the directional flow of the volume of gas between a first flow direction and a second flow direction within the gas delivery conduit (100).

11. The apparatus (10) of Claim 1, wherein the apparatus (10) is configured to be fluidly connected to a photoionization detector (150) and positioned upstream from the photoionization detector.

12. A method for reducing the humidity of a volume of gas, the method comprising:
passing a volume of gas through a gas delivery conduit (100) in a first flow direction, wherein the gas delivery conduit comprises a first conducting portion (110) and a second conducting portion (210), and wherein at least one heat-conductive media comprises a first heat-conductive media (111) positioned in-line within the first conducting portion of the gas delivery conduit (110) and a second heat-conductive media (211) positioned in-line within the second conducting portion (210) of the gas delivery conduit (100); and
cooling the first and second heat-conductive media within the first conducting portion (110) of the gas delivery conduit (100) and the second conducting portion (210) of the gas delivery conduit (100) via a cooler (130) in contact with an outer surface of the first conducting portion (110) of the gas delivery conduit (100) and the second conducting portion (210) of the gas delivery conduit (100) to condense humidity within a volume of gas on a surface of the first and second heat-conductive media.

13. The method of Claim 12, wherein the cooler (130) is a solid-state thermoelectric cooler and wherein cooling the at least one heat-conductive media comprises applying a voltage in a first direction to the cooler (130) to cool a first side of the cooler, and wherein the first side of the cooler (130) is in contact with the first conducting portion (110) of the gas delivery conduit (100).

14. The method of Claim 13, further comprising reversing the voltage applied to the cooler (130) to heat the first side (131) of the cooler (130) to heat the at least one heat-conductive media to remove condensed water from the gas delivery conduit.

15. The method of Claim 12, wherein the first conducting portion (110) of the gas delivery conduit is fluidly connected to a photoionization detector (150) and positioned upstream from the photoionization detector.

## Patentansprüche

1. Vorrichtung (10) zum Reduzieren der Feuchtigkeit eines Gasvolumens, wobei die Vorrichtung umfasst:
eine Gaszufuhrleitung (100), die einen ersten leitenden Abschnitt (110) und einen zweiten leitenden Abschnitt (210) umfasst;
mindestens ein wärmeleitfähiges Medium, das ein erstes wärmeleitfähiges Medium (111), das innerhalb des Abschnitts des ersten leitenden Abschnitts (110) der Gaszufuhrleitung (100) inline positioniert ist, und ein zweites wärmeleitfähiges Medium (211) umfasst, das innerhalb des zweiten leitenden Abschnitts (210) der Gaszufuhrleitung (100) inline positioniert ist; und
einen Kühler (130) in Kontakt mit dem ersten leitenden Abschnitt (110) und dem zweiten leitenden Abschnitt (210) der Gaszufuhrleitung (100), um den ersten leitenden Abschnitt (110) und den zweiten leitenden Abschnitt (210) der Gaszufuhrleitung und das erste und das zweite wärmeleitfähige Medium zu kühlen.

2. Vorrichtung (10) nach Anspruch 1, ferner umfassend ein Gehäuse (140), wobei das Gehäuse einen äußeren Gehäuseabschnitt und einen inneren Gehäuseabschnitt umfasst und wobei mindestens ein Abschnitt der Gaszufuhrleitung (100), das mindestens eine wärmeleitfähige Medium und der Kühler (130) innerhalb des inneren Gehäuseabschnitts umschlossen sind.

3. Vorrichtung (10) nach Anspruch 1, wobei das mindestens eine wärmeleitfähige Medium konfiguriert ist, um den Durchgang eines Gasvolumens dadurch zu ermöglichen.

4. Vorrichtung (10) nach Anspruch 1, wobei das mindestens eine wärmeleitfähige Medium ein Kupfermaterial umfasst.

5. Vorrichtung (10) nach Anspruch 1, wobei der erste und der zweite leitende Abschnitt (110, 210) wärmeleitfähige Seitenwände umfassen und wobei das erste und das zweite wärmeleitfähige Medium innerhalb eines inneren Abschnitts des ersten und des zweiten leitenden Abschnitts der Gaszufuhrleitung (100) positioniert sind.

6. Vorrichtung (10) nach Anspruch 1, wobei der Kühler (130) als ein thermoelektrischer Festkörperkühler ausgeführt ist.

7. Vorrichtung (10) nach Anspruch 6, wobei der Kühler (130) eine erste Seite (131) und eine zweite Seite (132) definiert und wobei auf wenigstens teilweise der Grundlage einer an den Kühler (130) angelegten Spannung die erste Seite (131) als Kühlseite und die zweite Seite (132) als Heizseite konfiguriert ist und wobei der mindestens eine Abschnitt der Gaszufuhrleitung (100) in Kontakt mit der ersten Seite steht.

8. Vorrichtung (10) nach Anspruch 7, wobei die Vorrichtung (10) konfiguriert ist, um die an den Kühler (130) angelegte Spannung selektiv umzukehren, sodass die erste Seite (131) als Heizseite konfiguriert ist und die zweite Seite (132) als Kühlseite konfiguriert ist.

9. Vorrichtung (10) nach Anspruch 8, wobei das erste wärmeleitfähige Medium (111) und ein zweites wärmeleitfähiges Medium (211) innerhalb eines inneren Abschnitts des ersten leitenden Abschnitts der Gaszufuhrleitung (110) bzw. des zweiten leitenden Abschnitts der Gaszufuhrleitung (210) positioniert sind und wobei der erste leitende Abschnitt der Gaszufuhrleitung (210) in Kontakt mit der ersten Seite des Kühlers (131) steht und der zweite leitende Abschnitt der Gaszufuhrleitung (210) in Kontakt mit der zweiten Seite des Kühlers (132) steht.

10. Vorrichtung (10) nach Anspruch 1, ferner umfassend eine Pumpe (160), die konfiguriert ist, um eine Strömung eines Gasvolumens durch die Gaszufuhrleitung (100) zu leiten und die Strömungsrichtung des Gasvolumens zwischen einer ersten Strömungsrichtung und einer zweiten Strömungsrichtung innerhalb der Gaszufuhrleitung (100) zu ändern.

11. Vorrichtung (10) nach Anspruch 1, wobei die Vorrichtung (10) konfiguriert ist, um mit einem Fotoionisationsdetektor (150) in Fluidverbindung zu stehen und in Strömungsrichtung dem Fotoionisationsdetektor vorgelagert positioniert zu sein.

12. Verfahren zum Reduzieren der Feuchtigkeit eines Gasvolumens, wobei das Verfahren umfasst:
Leiten eines Gasvolumens durch eine Gaszufuhrleitung (100) in einer ersten Strömungsrichtung, wobei die Gaszufuhrleitung einen ersten leitenden Abschnitt (110) und einen zweiten leitenden Abschnitt (210) umfasst und wobei mindestens ein wärmeleitfähiges Medium ein erstes wärmeleitfähiges Medium (111), das innerhalb des ersten leitenden Abschnitts der Gaszufuhrleitung (110) inline positioniert ist, und ein zweites wärmeleitfähiges Medium (211) umfasst, das innerhalb des zweiten leitenden Abschnitts (210) der Gaszufuhrleitung (100) inline positioniert ist; und
Kühlen des ersten und des zweiten wärmeleitfähigen Mediums innerhalb des ersten leitenden Abschnitts (110) der Gaszufuhrleitung (100) und des zweiten leitenden Abschnitts (210) der Gaszufuhrleitung (100) über einen Kühler (130) in Kontakt mit einer äußeren Oberfläche des ersten leitenden Abschnitts (110) der Gaszufuhrleitung (100) und des zweiten leitenden Abschnitts (210) der Gaszufuhrleitung (100), um Feuchtigkeit innerhalb eines Gasvolumens auf einer Oberfläche des ersten und des zweiten wärmeleitfähigen Mediums zu kondensieren.

13. Verfahren nach Anspruch 12, wobei der Kühler (130) ein thermoelektrischer Festkörperkühler ist und wobei das Kühlen des mindestens einen wärmeleitfähigen Mediums das Anlegen einer Spannung in einer ersten Richtung an den Kühler (130) umfasst, um eine erste Seite des Kühlers zu kühlen, und wobei die erste Seite des Kühlers (130) in Kontakt mit dem ersten leitenden Abschnitt (110) der Gaszufuhrleitung (100) steht.

14. Verfahren nach Anspruch 13, ferner umfassend das Umkehren der an den Kühler (130) angelegten Spannung, um die erste Seite (131) des Kühlers (130) zu erwärmen, um das mindestens eine wärmeleitfähige Medium zu erwärmen, um kondensiertes Wasser aus der Gaszufuhrleitung zu entfernen.

15. Verfahren nach Anspruch 12, wobei der erste leitende Abschnitt (110) der Gaszufuhrleitung in Fluidverbindung mit einem Fotoionisationsdetektor (150) steht und in Strömungsrichtung dem Fotoionisationsdetektor vorgelagert positioniert ist.

## Revendications

1. Appareil (10) destiné à réduire l'humidité d'un volume de gaz, l'appareil comprenant :
un conduit de distribution de gaz (100) et comprenant une première partie conductrice (110) et une seconde partie conductrice (210) ;
au moins un support conducteur de chaleur comprenant un premier support conducteur de chaleur (111) positionné en ligne dans la première partie conductrice (110) du conduit de distribution de gaz (100) et un second support conducteur de chaleur (211) positionné en ligne dans la seconde partie conductrice (210) du conduit de distribution de gaz (100) ; et
un refroidisseur (130) en contact avec la première partie conductrice (110) et la seconde partie conductrice (210) du conduit de distribution de gaz (100) pour refroidir la première partie conductrice (110) et la seconde partie conductrice (210) du conduit de distribution de gaz et les premier et second supports conducteurs de chaleur.

2. Appareil (10) selon la revendication 1, comprenant en outre un logement (140), dans lequel le logement comprend une partie de logement extérieure et une partie de logement intérieure, et dans lequel au moins une partie du conduit de distribution de gaz (100), l'au moins un support conducteur de chaleur et le refroidisseur (130) sont contenus dans la partie de logement intérieure.

3. Appareil (10) selon la revendication 1, dans lequel l'au moins un support conducteur de chaleur est configuré pour permettre le passage d'un volume de gaz à travers celui-ci.

4. Appareil (10) selon la revendication 1, dans lequel l'au moins un support conducteur de chaleur comprend un matériau en cuivre.

5. Appareil (10) selon la revendication 1, dans lequel les première et seconde parties conductrices (110, 210) comprennent des parois latérales conductrices de chaleur, et dans lequel des premier et second supports conducteurs de chaleur sont positionnés à l'intérieur d'une partie intérieure des première et seconde parties conductrices du conduit de distribution de gaz (100).

6. Appareil (10) selon la revendication 1, dans lequel le refroidisseur (130) est mis en oeuvre sous la forme d'un refroidisseur thermoélectrique à l'état solide.

7. Appareil (10) selon la revendication 6, dans lequel le refroidisseur (130) définit un premier côté (131) et un second côté (132), et dans lequel le premier côté (131) est configuré comme un côté de refroidissement et le second côté (132) est configuré comme un côté chauffant sur la base au moins en partie d'une tension appliquée aux bornes du refroidisseur (130), et dans lequel l'au moins une partie du conduit de distribution de gaz (100) est en contact avec le premier côté.

8. Appareil (10) selon la revendication 7, dans lequel l'appareil (10) est configuré pour inverser de manière sélective la tension appliquée aux bornes du refroidisseur (130) de telle sorte que le premier côté (131) est configuré à titre de côté chauffant et le second côté (132) est configuré à titre de côté de refroidissement.

9. Appareil (10) selon la revendication 8, dans lequel le premier support conducteur de chaleur (111) et un second support conducteur de chaleur (211) sont positionnés à l'intérieur d'une partie intérieure de la première partie conductrice du conduit de distribution de gaz (110) et de la seconde partie conductrice du conduit de distribution de gaz (210), respectivement, et dans lequel la première partie conductrice du conduit de distribution de gaz (210) est en contact avec le premier côté du refroidisseur (131) et la seconde partie conductrice du conduit de distribution de gaz (210) est en contact avec le second côté du refroidisseur (132).

10. Appareil (10) selon la revendication 1, comprenant en outre une pompe (160) configurée pour diriger un écoulement d'un volume de gaz à travers le conduit de distribution de gaz (100) et changer l'écoulement directionnel du volume de gaz entre une première direction d'écoulement et une seconde direction d'écoulement à l'intérieur du conduit de distribution de gaz (100).

11. Appareil (10) selon la revendication 1, dans lequel l'appareil (10) est configuré pour être relié fluidiquement à un détecteur de photoionisation (150) et positionné en mont du détecteur de photoionisation.

12. Procédé destiné à réduire l'humidité d'un volume de gaz, le procédé comprenant :
le passage d'un volume de gaz à travers un conduit de distribution de gaz (100) dans une première direction d'écoulement, le conduit de distribution de gaz comprenant une première partie conductrice (110) et une seconde partie conductrice (210), et dans lequel au moins un support conducteur de chaleur comprend un premier support conducteur de chaleur (111) positionné en ligne dans la première partie conductrice du conduit de distribution de gaz (110) et un second support conducteur de chaleur (211) positionné en ligne dans la seconde partie conductrice (210) du conduit de distribution de gaz (100) ; et
le refroidissement des premier et second supports conducteurs de chaleur à l'intérieur de la première partie conductrice (110) du conduit de distribution de gaz (100) et de la seconde partie conductrice (210) du conduit de distribution de gaz (100) via un refroidisseur (130) en contact avec une surface extérieure de la première partie conductrice (110) du conduit de distribution de gaz (100) et la seconde partie conductrice (210) du conduit de distribution de gaz (100) pour condenser l'humidité dans un volume de gaz sur une surface des premier et second supports conducteurs de chaleur.

13. Procédé selon la revendication 12, dans lequel le refroidisseur (130) est un refroidisseur thermoélectrique à l'état solide et dans lequel le refroidissement de l'au moins un support conducteur de chaleur comprend l'application d'une tension dans une première direction au refroidisseur (130) pour refroidir un premier côté du refroidisseur, et dans lequel le premier côté du refroidisseur (130) est en contact avec la première partie conductrice (110) du conduit de distribution de gaz (100).

14. Procédé selon la revendication 13, comprenant en outre une inversion de la tension appliquée au refroidisseur (130) pour chauffer le premier côté (131) du refroidisseur (130) afin de chauffer l'au moins un support conducteur de chaleur pour éliminer l'eau condensée du conduit de distribution de gaz.

15. Procédé selon la revendication 12, dans lequel la première partie conductrice (110) du conduit de distribution de gaz est reliée fluidiquement à un détecteur de photoionisation (150) et positionnée en amont du détecteur de photoionisation.
